# EUROPEAN PATENT SPECIFICATION

(11) **EP 1 729 811 B1**
(45) Date of publication and mention of the grant of the patent: **28.04.2010**
(21) Application number: 05732385.9
(22) Date of filing: 17.03.2005
(51) Int. Cl.: A61K 47/32, A61K 47/44, A61K 31/7048, A61K 9/107

(54) **CREAM-GEL CONTAINING IVERMECTIN**
IVERMECTIN ENTHALTENDE CREME
GEL-CREME CONTENANT DE L'IVERMECTINE

(30) Priority: 18.03.2004 FR 0402798; 25.03.2004 US 556028 P
(43) Date of publication of application: 13.12.2006
(73) Proprietor: Galderma S.A., 6330 Cham (CH)
(72) Inventor: ASTRUC, Fanny, F-06200 Nice (FR); ORSONI, Sandrine, 06210 Mandelieu (FR); FREDON, Laurent, Chem. du Camouyer, 06330 Roquefort les Pins (FR)
(74) Representative: Allab, Myriam
(86) International application number: PCT/EP2005/003718
(87) International publication number: WO 2005/089806

(56) References cited:
- WO-A-03/032976
- WO-A-03/032977
- WO-A-2004/093886
- FR-A- 2 854 074
- US-A- 5 952 372
- GALDERMA LABORATORIES: "Cetaphil cleansers and moisturizers"[Online] 2004, XP002287576 Retrieved from the Internet: URL:http://www.cetaphil.com>

## Description

The present invention relates to a pharmaceutical composition based on a compound of the avermectin family, in the form of a cream-gel comprising, in a physiologically acceptable medium, an oily phase dispersed in an aqueous phase by means of a non-surfactant polymeric emulsifier, said oily phase comprising oils having a melting point below 30°C and being free of solid fats having a melting point above 30°C.

It also relates to the process for preparing same and to its use in the production of a pharmaceutical preparation intended for the treatment of dermatological conditions, in particular of rosacea.

Ivermectin is a mixture of two compounds belonging to the avermectin class, 5-O-demethyl-22,23-dihydroavermectin A₁ₐ and 5-O-demethyl-22,23-dihydroavermectin A_{1b}. They are also known as 22,23-dihydroavermectin B₁ₐ and 22,23-dihydroavermectin B_{1b}. Ivermectin contains at least 80% of 22,23-dihydroavermectin B₁ₐ and less than 20% of 22,23-dihydroavermectin B_{1b}. This active agent is part of the avermectin class, a group of macrocyclic lactones produced by the bacterium *Streptomyces avermitilis* (Reynolds JEF (Ed) (1993) Marindale. The extra pharmacopoeia. 29th Edition. Pharmaceutical Press, London). The avermectins include, in particular, ivermectin, invermectin, avermectin, abamectin, doramectin, eprinomectin and selamectin.

Ivermectin is more particularly an antihelmintic. It has already been described in humans in the treatment of river blindness caused by Onchocerca volvulus, of gastrointestine strongyloidosis (anguillulosis) (product Stromectol®), and of human scabies (Meinking TL et al., N Engl J Med 1995 Jul 6;333(1):26-30 The treatment of scabies with ivermectin) and also in the treatment of microfilaremia diagnosed or suspected in individuals suffering from lymphatic filariasis due to Wuchereria bancrofti.

US patent 6,133,310 discloses the use of ivermectin topically in the form of a lotion consisting of a mixture of ivermectin and water, and also mentions the possibility of a cream consisting, for its part, of a mixture of ivermectin and an excipient such as propylene glycol or sodium lauryl sulphate, but describes no pharmaceutical composition that is industrially acceptable, i.e. having good cosmeticity and a sufficiently long shelf-life (minimum of 2 years).

Dermatological conditions are often associated with increased sensitivity of the skin, particularly in the case of rosacea, which is an inflammatory dermatosis that affects mainly the central part of the face and is characterized, inter alia, by reddening of the face, hot flushes, and facial erythema. This type of pathology requires particularly the use of pharmaceutical formulations that are easy to spread and give the user a pleasant feeling of well-being.

There exists therefore a need to have a topical pharmaceutical composition containing at least one compound of the avermectin family, and more particularly ivermectin, which is completely suitable for the pathology and specifically for sensitive skin, which is industrially acceptable, i.e. the formulation of which is physically stable (without phase separation) and chemically stable (without modification of the stability of the active agent), and which optimizes the penetration of ivermectin into the skin.

Now, the applicant has developed an ivermectin-based composition in the form of a cream-gel that corresponds completely to these expectations, comprising an oily phase dispersed in an aqueous phase by means of a non-surfactant polymeric emulsifier, said oily phase comprising oils having a melting point below 30°C and being free of solid fats having a melting point above 30°C.

This cream-gel formulation is a moisturizing emulsion with a non-greasy texture that is very well tolerated and that contains, as emulsifier, a non-surfactant polymer. This formulation also combines the advantages of a gel (ease of application, rapid release of the active agent, freshness on application) with those of a cream (comfort of the skin, no dryness or tightness that is completely unacceptable for sensitive skin) and is particularly suitable for the treatment of rosacea.

Conventional emulsions as described in the prior art are virtually homogeneous unstable systems of two immiscible liquids, one of which is dispersed in the other in the form of fine droplets (micelles). This dispersion is stabilized by means of the action of surfactant emulsifiers which modify the structure and the ratio of the forces at the interface, and therefore increase the stability of the dispersion by decreasing the surface tension energy.

Surfactant emulsifiers are amphiphilic compounds that have a hydrophobic portion with affinity for the oil and a hydrophilic portion with affinity for the water, thus creating a link between the two phases. Ionic or nonionic emulsifiers therefore stabilize oil/water emulsions by adsorbing at the interface and forming lamellar layers of liquid crystals.

The emulsifying power of nonionic surfactants is closely linked to the polarity of the molecule. This polarity is defined by the HLB (hydrophilic/lipophilic balance). Conventional emulsions are generally stabilized by a mixture of surfactants, the HLBs of which can be quite different, but the proportion of which in the mixture corresponds to the required HLB of the fatty phase to be emulsified. This type of surfactant emulsifier is often used at a concentration of between 3 and 7%.

Non-surfactant polymeric emulsifiers such as acrylate/C₁₀₋₃₀ alkyl acrylate crosspolymers make it possible to prepare virtually homogeneous systems (oily phase dispersed in an aqueous phase) called polymer emulsions. The polymer emulsification is obtained by steric stabilization: the globules of dispersed phase (oil) are surrounded by hydrophilic polymer that is anchored therein by virtue of a grafted hydrophobic chain.

The crystalline phase is absent and this type of emulsifier does not affect the surface tension, unlike surfactant emulsifiers. These non-surfactant polymeric emulsifiers are hydrophilic compounds that are not characterized by an HLB value and do not form micelles. It is in this respect that polymer emulsions are also called "emulsifier-free systems". These emulsifiers are effective from a concentration of 0.1%.

Moreover, the use, in the oily phase, of the composition of liquid oils having a melting point below 30°C makes it possible to obtain a fluid cream-gel that is light and has a non-greasy texture on application, and facilitates the preparation of the cream-gel, which can also be produced at ambient temperature.

By virtue of its composition, this cream-gel therefore guarantees that the composition is both stable and innocuous. It is easier to spread than a conventional emulsion due to its gelled structure and its "quick-break" effect, and leaves a pleasant feeling of freshness.

The "quick-break" effect, **characterized in that** the emulsion "breaks" immediately and releases the aqueous and oily phases, is due to the sensitivity of acrylic acid homopolymers and copolymers to electrolytes. When an emulsion containing this type of polymeric emulsifier is applied to the skin, the polymers come into contact with the salts present thereon. This "quick-break" effect promotes faster release of the phase containing the active agent.

The applicant has therefore discovered, surprisingly, that the cream-gel composition according to the invention also allows better release of the active principle and better penetration thereof into the skin, compared with the conventional emulsions.

A subject of the invention is therefore a pharmaceutical composition based on a compound of the avermectin family, in the form of a cream-gel comprising, in a physiologically acceptable medium, an oily phase dispersed in an aqueous phase by means of a non-surfactant polymeric emulsifier, said oily phase comprising oils having a melting point below 30°C and being free of solid fats having a melting point above 30°C.

The compounds of the avermectin family are chosen from ivermectin, invermectin, avermectin, abamectin, doramectin, eprinomectin and selamectin, and preferably ivermectin.

A subject of the invention is also an ivermectin-based pharmaceutical composition in the form of a cream-gel comprising, in a physiologically acceptable medium, an oily phase dispersed in an aqueous phase by means of a non-surfactant polymeric emulsifier, said oily phase comprising oils having a melting point below 30°C and being free of solid fats having a melting point above 30°C.

The invention can also be applied to any active agent chosen from the avermectin family, and in particular avermectin, abamectin, doramectin, eprinomectin and selamectin, and mixtures thereof.

The term "physiologically acceptable medium" is intended to mean a medium that is compatible with the skin, the mucous membranes, the lips, the nails, the scalp and/or the hair.

The expression "oils having a melting point below 30°C" is intended to mean oils that are in the liquid state at ambient temperature.

The term "solid fats" is intended to mean in particular waxes, fatty acids and fatty alcohols having a melting point above 30°C, that are in the solid state at ambient temperature.

The term "free of solid fats" according to the invention is intended to mean a composition comprising less than 1% of solid fats, preferably less than 0.1%, even more preferably less than 0.05% of solid fats.

The composition according to the invention is described as a stable emulsion in that it has good physical and chemical stability over time, even at a temperature above ambient temperature (for example 45-55°C), as shown by the examples described below.

The composition according to the invention is advantageously a cream-gel which comprises:
a) an oily phase comprising oils having a melting point below 30°C and being free of solid fats having a melting point above 30°C,
b) a non-surfactant polymeric emulsifier,
c) at least one compound of the avermectin family,
d) a solvent and/or a propenetrating agent for the active agent,
e) and water.

The composition according to the invention is preferably a cream-gel which comprises:
a) an oily phase comprising oils having a melting point below 30°C and being free of solid fats having a melting point above 30°C,
b) a non-surfactant polymeric emulsifier,
c) ivermectin,
d) a solvent and/or propenetrating agent for the active agent,
e) and water.

The oily phase therefore comprises liquid oils, among which mention may be made, for example, of plant, mineral, animal or synthetic oils, silicone oils, Guerbet alcohols, and mixtures thereof; an apolar mineral oil (high surface tension) of the paraffin oil type will preferably be used.

As an example of a mineral oil, mention may be made, for example, of Primol 352, Marcol 82 and Marcol 152, sold by the company Esso.

As a plant oil, mention may be made of sweet almond oil, palm oil, soybean oil, sesame oil, sunflower oil, an ester such as cetaryl isononanoate, for instance the product sold under the name Cetiol SN by the company Cognis France, diisopropyl adipate, for instance the product sold under the name Ceraphyl 230 by the company ISF, isopropyl palmitate, for instance the product sold under the name Crodamol IPP by the company Croda, or caprylic/capric triglyceride such as Miglyol 812 sold by the company Huls/Lambert Rivière.

As a silicone oil, mention may be made of a dimethicone, for instance the product sold under the name Dow Corning 200 fluid, or a cyclomethicone, for instance the product sold under the name Dow Corning 244 fluid by the company Dow Corning or the product sold under the name Mirasil CM5 by the company SACI-CFPA.

The amount of oily phase present in the composition generally ranges from 4 to 60%, and preferably from 5 to 20%, and more precisely from 8 to 12%, by weight relative to the total weight of the composition.

According to the invention, the term "non-surfactant polymeric emulsifier" is intended to mean an emulsifier system comprising at least one copolymer consisting of a major fraction of monomer of monoolefinically unsaturated C₃-C₆ carboxylic acid or of its anhydride, and of a minor fraction of monomer of acrylic acid ester containing a fatty chain.

The emulsifying copolymers of the invention are described in patent application EP-A-0268164 and are obtained according to the preparation methods described in that same document.

Use is more particularly made of the acrylate/C₁₀-C₃₀ alkyl acrylate copolymer, such as the products sold under the names Pemulen TR 1 and Pemulen TR 2 or the product sold under the name Carbopol 1342 and Carbopol 1382 by the company Goodrich, or else mixtures thereof.

Preferably, the composition according to the invention comprises a non-surfactant polymeric emulsifier chosen from Pemulen TR1 and Pemulen TR2.

The amount of non-surfactant polymeric emulsifiers in the composition according to the invention is generally between 0.25% and 2%, preferably from 0.25 to 1%, and even more preferably from 0.3 to 0.6%, by weight relative to the total weight of the composition.

The composition according to the invention contains from 0.001 to 10% of ivermectin by weight relative to the total weight of the composition. Preferably, the composition comprises from 0.01 to 5% by weight relative to the total weight of the composition, even more preferably from 0.01 to 1% by weight relative to the total weight of the composition.

By way of example of a solvent and/or a propenetrating agent for the ivermectin active agent, mention will preferably be made of propylene glycol, alcohols such as ethanol, isopropanol, butanol, benzyl alcohol, N-methyl-2-pyrrolidone or DMSO, polysorbate 80, phenoxyethanol, and mixtures thereof.

| Solvents | Maximum % solubility of ivermectin in the solvent under consideration (weight/weight) |
|---|---|
| N-methyl-2-pyrrolidone | 58.13 |
| Propylene glycol/oleic acid (4 parts/2 parts) | 27.31 |
| Propylene glycol/polysorbate 80/ phenoxyethanol (4 parts/1 part/1 part) | 28.63 |

The composition of the invention contains from 0.1% to 20%, and preferably from 1% to 10%, of a solvent and/or a propenetrating agent for the ivermectin active agent.

The composition of the invention also contains water ranging from 30 to 95%, and preferably from 60 to 80%, by weight relative to the total weight of the composition. The water used in the composition according to the invention will preferably be purified water.

The composition according to the invention may also comprise a carbomer ranging from 0 to 2%. Among the carbomers, mention may be made, by way of non-limiting examples, of Carbopol 981, Carbopol ETD 2020, Carbopol 980 or Carbopol Ultrez 10 NF, sold by the company BF Goodrich.

The composition according to the invention may also comprise co-emulsifiers in order to decrease the size of the oily globules of the emulsion formed by the polymeric emulsifier. Mention may be made of nonionic surfactants such as Eumulgin B2 (ceteareth 20) or Tween 80 (polysorbate 80) or else sorbitan esters (including span 20) or fatty alcohol ethers (including Eumulgin B2) or poloxamer 124.

Preferably, the cream-gel based on a compound of the avermectin family according to the invention comprises:
5 to 20% of oily phase,
0.25 to 1% of non-surfactant polymeric emulsifier,
0 to 2% of carbomer,
0 to 5% of co-emulsifier,
0.01 to 5% of at least one avermectin,
0.1 to 20% of solvents and/or propenetrating agents,
60 to 80% of water.

Preferably, the invermectin-based cream-gel according to the invention comprises:
5 to 20% of oily phase,
0.25 to 1% of non-surfactant polymeric emulsifier,
0 to 2% of carbomer,
0 to 5% of co-emulsifier,
0.01 to 5% of ivermectin,
0.1 to 20% of solvents and/or propenetrating agents,
60 to 80% of water.

More preferably, the invermectin-based cream-gel according to the invention comprises:
8 to 15% of oily phase,
0.25 to 1% of non-surfactant polymeric emulsifier,
0 to 2% of carbomer,
1 to 5% of co-emulsifier,
0.01 to 5% of ivermectin,
4 to 20% of solvent and/or propenetrating agents,
60 to 80% of water.

The composition according to the invention may also contain additives normally used in the cosmetics or pharmaceutical field, such as
- humectants, such as glycerol or sorbitol;
- gelling agents;
- calmatives such as allantoin and talc;
- preserving agents such as para-hydroxybenzoic acid esters;
- moisture regulators;
- pH regulators such as citric acid and sodium hydroxide;
- osmotic pressure modifiers;
- UV-A and UV-B screening agents;
- and antioxidants, such as α-tocopherol, butylhydroxyanisole (BHA) or butylhydroxytoluene (BHT), vitamin E, propyl gallate or citric acid.

Of course, those skilled in the art will be able to adapt the choice of additives or the optional compounds to be added to these compositions and also the procedure in such a way that the advantageous properties intrinsically associated with the present invention are not, are not substantially, impaired by the envisaged addition.

These additives may be present in the composition at from 0.001 to 20% by weight relative to the total weight of the composition.

The cream-gel according to the invention will preferably have a viscosity corresponding to a flow threshold ranging from 15 to 60 Pascal (Pa), in particular from 20 to 50 Pa, measured using a Haake VT500-type rheometer.

The composition according to the invention can be prepared according to a "hot" (60-70°C) or "cold" (ambient temperature) embodiment, according to the nature of the additives used.

According to a particular embodiment, the process for preparing the cream-gel according to the invention is carried out at ambient temperature (referred to as "cold") and comprises, successively, the following steps:
a) the constituents of the fatty phase are mixed until said phase is homogeneous;
b) the constituents of the aqueous phase are dissolved in water until complete homogeneity is obtained;
c) the non-surfactant polymeric emulsifier and the optional carbomer are dispersed in the aqueous phase obtained in b) until a homogeneous gel is obtained;
d) the fatty phase is incorporated, with moderate mechanical stirring, into the homogeneous gel obtained in c) so as to form an emulsion;
e) the constituents of active phase are mixed; the ivermectin is solubilized in this mixture; this phase is then incorporated into the emulsion obtained in d), with moderate mechanical stirring;
f) the neutralizing agent is incorporated at the end of step e) or during one of the preceding steps with moderate mechanical stirring so as to obtain a defined pH.

Preferably, this pH will be between 6.0 and 6.5. Verification of the natural pH of the mixture and optional correction with a solution of a neutralizing agent, and also incorporation of the optional additives, may be carried out, according to their chemical nature, during one of the steps of the preparation process described above.

The term "moderate mechanical stirring" used during the preparation process according to the invention is intended in particular to mean a mechanical stirring of between 500 and 1200 rpm measured with a Rayneri stirrer, and more preferably of between 600 and 1000 rpm.

A subject of the invention is also the use of the composition according to the invention, for producing a pharmaceutical preparation intended to treat dermatological conditions.

The term "dermatological conditions" is intended to mean more particularly rosacea, common acne, seborrhoeic dermatitis, perioral dermatitis, acneform eruptions, transient acantholytic dermatitis, and acne miliaris *necrotica.*

The composition according to the invention is particularly suitable for the treatment of rosacea.

Various formulations of cream-gel type comprising ivermectin according to the invention will now be given by way of illustration that is in no way limiting in nature. The amounts therein are given as % by weight, unless otherwise mentioned.

### EXAMPLES

### Example 1:

| | |
|---|---|
| Ivermectin | 1.00 |
| Mineral oil | 10.00 |
| Tocopherol | 0.20 |
| Propyl paraben | 0.10 |
| Disodium edetate | 0.10 |
| Glycerol | 5.00 |
| Allantoin | 0.20 |
| Acrylates/C₁₀₋₃₀ alkyl acrylate | |
| crosspolymer (Pemulen TR1) | 0.30 |
| Carbomer | 0.15 |
| Polysorbate 80 | 4.00 |
| Propylene glycol | 4.00 |
| Phenoxyethanol | 1.00 |
| | qs pH |
| Sodium hydroxide | 6.30 |
| Purified water | qs 100 |

This cream-gel can be prepared according to the preparation process carried out at ambient temperature ("cold") comprising the following steps:
a) the mineral oil, the tocopherol and the propyl paraben are mixed until complete homogeneity is obtained;
b) the glycerol, the allantoin and the sodium edetate are dissolved in the water until complete homogeneity is obtained;
c) the acrylates/C₁₀₋₃₀ alkyl acrylate crosspolymer and the carbomer are dispersed in the aqueous phase previously obtained in step b), until a homogeneous gel is obtained;
d) the fatty phase obtained in step a) is incorporated, with moderate mechanical stirring (Rayneri: 1000 rpm), into the homogeneous gel obtained in step c);
e) the polysorbate 80, the propylene glycol and the phenoxyethanol are mixed; the ivermectin is solubilized in this mixture; this phase is then incorporated into the emulsion obtained in step d), with moderate mechanical stirring (Rayneri: approximately 600 rpm);

The neutralizing agent is incorporated, with moderate mechanical stirring (Rayneri: approximately 600 rpm), so as to obtain pH 6.30.

### Example 2:

| | |
|---|---|
| Ivermectin | 1.00 |
| Mineral oil | 10.00 |
| Tocopherol | 0.20 |
| Disodium edetate | 0.10 |
| Glycerol | 5.00 |
| Acrylates/C₁₀₋₃₀ alkyl acrylate | |
| crosspolymer (Pemulen TR1) | 0.30 |
| Carbomer | 0.15 |
| Polysorbate 80 | 4.00 |
| Propylene glycol | 4.00 |
| Phenoxyethanol | 1.00 |
| | qs pH |
| Triethanolamine | 6.00 |
| Purified water | qs 100 |

### Example 3:

| | |
|---|---|
| Ivermectin | 0.50 |
| Capric/caprylic triglycerides | 10.00 |
| Tocopherol | 0.20 |
| Disodium edetate | 0.10 |
| Sorbitol | 5.00 |
| Zinc acetate | 0.50 |
| Acrylates/C₁₀₋₃₀ alkyl acrylate | |
| crosspolymer (Pemulen TR1) | 0.30 |
| Carbomer | 0.15 |
| N-methylpyrrolidone | 5.00 |
| Phenoxyethanol | 1.00 |
| | qs pH |
| Sodium hydroxide | 6.00 |
| Purified water | qs 100 |

### Example 4:

| | |
|---|---|
| Ivermectin | 1.00 |
| Mineral oil | 10.00 |
| Sorbitan laurate | 1.00 |
| Tocopherol | 0.20 |
| Propyl paraben | 0.10 |
| Disodium edetate | 0.10 |
| Glycerol | 5.00 |
| Allantoin | 0.20 |
| Acrylates/C₁₀₋₃₀ alkyl acrylate | |
| crosspolymer (Pemulen TR1) | 0.30 |
| Carbomer | 0.15 |
| Polysorbate 80 | 3.00 |
| Poloxamer 124 | 1.00 |
| Propylene glycol | 4.00 |
| Phenoxyethanol | 1.00 |
| | qs pH 6.3 |
| Sodium hydroxide | 6.00 |
| Purified water | qs 100 |

### Example 5:

| | |
|---|---|
| Ivermectin | 1.00 |
| Mineral oil | 10.00 |
| Sorbitan laurate | 1.00 |
| Tocopherol | 0.20 |
| Propyl paraben | 0.10 |
| Disodium edetate | 0.10 |
| Glycerol | 5.00 |
| Allantoin | 0.20 |
| Acrylates/C₁₀₋₃₀ alkyl acrylate | |
| crosspolymer (Pemulen TR1) | 0.30 |
| Carbomer | 0.15 |
| Polysorbate 80 | 4.00 |
| Propylene glycol | 4.00 |
| Benzyl alcohol | 3.00 |
| | qs pH 6.3 |
| Sodium hydroxide | 6.00 |
| Purified water | qs 100 |

### Example 6:

| | |
|---|---|
| Ivermectin | 0.03 |
| Mineral oil | 10.00 |
| Sorbitan laurate | 1.00 |
| Tocopherol | 0.20 |
| Propyl paraben | 0.10 |
| Disodium edetate | 0.10 |
| Glycerol | 5.00 |
| Allantoin | 0.20 |
| Acrylates/C₁₀₋₃₀ alkyl acrylate | |
| crosspolymer (Pemulen TR1) | 0.30 |
| Carbomer | 0.15 |
| Polysorbate 80 | 4.00 |
| Propylene glycol | 4.00 |
| Benzyl alcohol | 3.00 |
| Poly(methyl methacrylate) | 2.00 |
| | qs pH 6.3 |
| Sodium hydroxide | 6.00 |
| Purified water | qs 100 |

### Example 7:

| | |
|---|---|
| Ivermectin | 0.03 |
| Mineral oil | 5.00 |
| Sweet almond oil | 5.00 |
| Sorbitan laurate | 1.00 |
| Tocopherol | 0.20 |
| Propyl paraben | 0.10 |
| Disodium edetate | 0.10 |
| Glycerol | 5.00 |
| Allantoin | 0.20 |
| Acrylates/C₁₀₋₃₀ alkyl acrylate | |
| crosspolymer (Pemulen TR1) | 0.30 |
| Carbomer | 0.15 |
| Polysorbate 80 | 4.00 |
| Propylene glycol | 4.00 |
| Benzyl alcohol | 3.00 |
| | qs pH 6.3 |
| Sodium hydroxide | 6.00 |
| Purified water | qs 100 |

### Example 8:

| | |
|---|---|
| Eprinomectin | 1.00 |
| Mineral oil | 10.00 |
| Tocopherol | 0.20 |
| Propyl paraben | 0.10 |
| Disodium edetate | 0.10 |
| Glycerol | 5.00 |
| Allantoin | 0.20 |
| Acrylates/C₁₀₋₃₀ alkyl acrylate | |
| crosspolymer (Pemulen TR1) | 0.30 |
| Carbomer | 0.15 |
| Polysorbate 80 | 4.00 |
| Propylene glycol | 4.00 |
| Phenoxyethanol | 1.00 |
| | qs pH |
| Sodium hydroxide | 6.30 |
| Purified water | qs 100 |

These compositions are intended to be applied daily to a clean and dry skin. Patients suffering from rosacea note a relief from the first applications and an improvement in the rosacea blotches is observed from 10 days of treatment.

### Example 9: Chemical and physical stability of the formulations

### a) pH and chemical stability

The variation in pH of the formulations is measured at 4, 8, 10 and 12 weeks at ambient temperature (25°C) and at 45°C.

| Examples | Condition | Initial pH | pH week 4 | pH week 8 | pH week 10 | pH week 12 |
|---|---|---|---|---|---|---|
| 1 | 25°C/60% RH | 6.3 | 6.4 | 6.4 | 6.3 | 6.3 |
| | 45°C/75% RH | 6.3 | 6.2 | 6.2 | 6.2 | 6.2 |
| 2 | 25°C/60% RH | 6.0 | 6.1 | 6.0 | 6.0 | 6.1 |
| | 45°C/75% RH | 6.0 | 6.0 | 6.1 | 6.0 | 6.1 |

| | | | | | | |
|---|---|---|---|---|---|---|
| 60% RH = 60% relative humidity 75% RH = 75% relative humidity | | | | | | |

These results therefore show that the formulations described are stable over time with respect to pH, whether at ambient temperature or at 45°C.

The chemical stability is an assay of the active agent by HPLC at ambient temperature and 45°C, after 4, 8 and 12 weeks.

| Examples | Condition | Initial test | Test week 4 | Test week 8 | Test week 12 |
|---|---|---|---|---|---|
| 1 | 25°C/60% RH | 100.3 | 98.7 | 100.0 | 102.5 |
| | 45°C/75% RH | 100.3 | 100.5 | 102.3 | 106.1 |
| 2 | 25°C/60% RH | 95.7 | 97.1 | 98.4 | 96.8 |
| | 45°C/75% PH | 95.7 | 98.1 | 96.9 | 96.3 |

The results show that the active agent is stable in the composition and does not degrade over time whatever the temperature at which the product is stored.

### b) Physical stability

The physical stability of the formulations is measured by microscopic and macroscopic observation of the formulation at ambient temperature, at 4°C and at 45-55°C after 4, 8, 10, 12 and 16 weeks.

At ambient temperature, the macroscopic observation makes it possible to guarantee the physical integrity of the products and the microscopic observation makes it possible to verify that there is no recrystallization of the solubilized active agent and no significant change in the size of the globules of the emulsion.

At 4°C, the microscopic observation verifies the non-recrystallization of the solubilized active agent.

At 45-55°C, the macroscopic observation verifies the integrity of the finished product.

The formulations described in examples 1 and 2 were tested. No recrystallization of the product nor any phenomenon of phase separation (dephasing) is observed over time, whether at ambient temperature, at 4°C or at 45-55°C.

The formulations as described in the examples are therefore chemically and physically stable.

### Example 10: Measurement of viscosity of the formulations

A Haake VT500-type rheometer with an SVDIN measuring sensor.

The rheograms were determined at 25°C and at a shear rate of 4 s⁻¹ (γ), and by measuring the shear strain.

The term "flow threshold" (τ0 expressed in Pascals) is intended to mean the force necessary (minimum shear strain) to overcome the Van der Waals-type cohesion forces and cause flow. The flow threshold is comparable to the value found at the shear rate of 4 s⁻¹.

These measurements are taken at T0 and after 4, 8 and 12 weeks.

| Example | Flow threshold (τ0 expressed in Pa) | | | |
|---|---|---|---|---|
| | T0 | 4 weeks | 8 weeks | 12 weeks |
| 1 | 20 | 20 | 46* | 44* |
| 2 | 26 | 26 | 22 | 23 |

| | | | | |
|---|---|---|---|---|
| *: Use of a Haake VT 550 rheometer that induces a slight increase in the characteristic viscosity values. | | | | |

The results obtained show no significant variation in the flow threshold; the viscosity of the composition according to the invention is therefore stable over time.

### Example 11: Release-penetration

A study was carried out in order to compare the release-penetration of ivermectin at 1% incorporated into compositions of the conventional emulsion (A) or cream-gel (example 1) type, applied non-occlusively to samples of human skin. The tritium-radiolabelled compositions that were compared are a conventional emulsion formula A and example 1 in accordance with the invention.

Formula A (conventional emulsion) is as follows:

| Ingredients | % by weight relative to the total weight of the composition |
|---|---|
| Ivermectin | 1.00 |
| Glycerol | 4.0 |
| Steareth-2 | 1.0 |
| Steareth-21 | 2.0 |
| Aluminium magnesium silicate/titanium dioxide/silica | 1.0 |
| Methyl para-hydroxybenzoate | 0.2 |
| Propyl para-hydroxybenzoate | 0.1 |
| Disodium EDTA | 0.05 |
| Citric acid monohydrate | 0.05 |
| Isopropyl palmitate | 4.0 |
| Glyceryl/PEG 100 stearate | 2.0 |
| Self-emulsifiable wax | 1.0 |
| Palmitostearic acid | 2.00 |
| Dimethicone 200-350 cS | 0.5 |
| Propylene glycol | 4.0 |
| Glycerol triacetate | 1.00 |
| Phenoxyethanol | 0.5 |
| 10% sodium hydroxide | qs |
| Water | qs 100 |

The compositions are applied, for 16 hours, to the human skin samples placed on continuous-flow automatic diffusion cells (Scott/Dick, University of Newcastle-upon-Tyne, UK).

The radioactivity is then measured in the various layers of the skin and in the recipient liquid in contact with the skin sample.

| Composition | % total radioactivity recovered |
|---|---|
| Formula A | 2.10 |
| Example 1 | 3.28 |

The results obtained clearly show that the composition in the form of a cream-gel described in example 1 substantially and significantly increases the penetration of ivermectin into the various layers of the skin.

## Claims

1. Pharmaceutical composition based on a compound of the avermectin family, in the form of a cream-gel comprising, in a physiologically acceptable medium, an oily phase dispersed in an aqueous phase by means of a non-surfactant polymeric emulsifier, said oily phase comprising oils having a melting point below 30°C and being free of solid fats having a melting point above 30°C.

2. Pharmaceutical composition according to Claim 1, **characterized in that** the compound of the avermectin family is chosen from ivermectin, invermectin, avermectin, abamectin, doramectin, eprinomectin and selamectin.

3. Pharmaceutical composition according to either of Claims 1 and 2, **characterized in that** the compound of the avermectin family is ivermectin.

4. Composition according to Claim 3, **characterized in that** it comprises:
a) an oily phase composed of oils having a melting point below 30°C and being free of solid fats having a melting point above 30°C,
b) a non-surfactant polymeric emulsifier,
c) ivermectin,
d) a solvent and/or propenetrating agent for the active agent,
e) and water.

5. Composition according to either of Claims 3 and 4, **characterized in that** the amount of ivermectin represents from 0.01 to 10%, and preferably from 0.01 to 5%, by weight relative to the total weight of the composition.

6. Composition according to one of Claims 1 to 5, **characterized in that** the non-surfactant polymeric emulsifier is chosen from Pemulen TR 1, Pemulen TR 2, Carbopol 1342, Carbopol 1382 or mixtures thereof.

7. Composition according to Claim 4, **characterized in that** the solvent and/or propenetrating agent is chosen from propylene glycol, ethanol, isopropanol, butanol, benzyl alcohol, N-methyl-2-pyrrolidone or DMSO, polysorbate 80, phenoxyethanol, and mixtures thereof.

8. Composition according to one of Claims 1 to 7, **characterized in that** it contains an amount of water ranging from 30 to 95% by weight relative to the total weight of the composition.

9. Composition according to one of Claims 1 to 8, **characterized in that** it comprises:
5 to 20% of oily phase,
0.25 to 1% of non-surfactant polymeric emulsifier,
0 to 2% of carbomer,
0 to 5% of co-emulsifier,
0.01 to 5% of ivermectin,
0.1 to 20% of solvents and/or propenetrating agents,
60 to 80% of purified water.

10. Composition according to one of Claims 1 to 9, **characterized in that** it comprises:
8 to 15% of oily phase,
0.25 to 1% of non-surfactant polymeric emulsifier,
0 to 2% of carbomer,
1 to 5% of co-emulsifier,
0.01 to 5% of ivermectin,
4 to 20% of solvents and/or propenetrating agents,
60 to 80% of purified water.

11. Composition according to one of Claims 1 to 10, **characterized in that** it comprises:
| | |
|---|---|
| Ivermectin | 1.00% |
| Mineral oil | 10.00% |
| Tocopherol | 0.20% |
| Propyl paraben | 0.10% |
| Disodium edetate | 0.10% |
| Glycerol | 5.00% |
| Allantoin | 0.20% |
| Acrylates/C₁₀₋₃₀ alkyl acrylate | |
| crosspolymer | 0.30% |
| Carbomer | 0.15% |
| Polysorbate 80 | 4.00% |
| Propylene glycol | 4.00% |
| Phenoxyethanol | 1.00 % |
| Sodium hydroxide | qs pH 6.30 |
| Purified water | qs 100 |

12. Composition according to one of Claims 1 to 10, **characterized in that** it comprises:
| | |
|---|---|
| Ivermectin | 1.00% |
| Mineral oil | 10.00% |
| Tocopherol | 0.20% |
| Disodium edetate | 0.10% |
| Glycerol | 5.00% |
| Acrylates/C₁₀₋₃₀ alkyl acrylate | |
| crosspolymer | 0.30% |
| Carbomer | 0.15% |
| Polysorbate 80 | 4.00% |
| Propylene glycol | 4.00% |
| Phenoxyethanol | 1.00% |
| Triethanolamine | qs pH 6.00 |
| Purified water | qs 100% |

13. Composition according to one of Claims 1 to 10, **characterized in that** it comprises:
| | |
|---|---|
| Ivermectin | 0.50% |
| Capric/caprylic triglycerides | 10.00% |
| Tocopherol | 0.20% |
| Disodium edetate | 0.10% |
| Sorbitol | 5.00% |
| Zinc acetate | 0.50% |
| Acrylates/C₁₀₋₃₀ alkyl acrylate | |
| crosspolymer | 0.30% |
| Carbomer | 0.15% |
| N-methylpyrrolidone | 5.00% |
| Phenoxyethanol | 1.00% |
| Sodium hydroxide | qs pH 6.00 |
| Purified water | qs 100% |

14. Composition according to one of Claims 1 to 10, **characterized in that** it comprises:
| | |
|---|---|
| Ivermectin | 1.00% |
| Mineral oil | 10.00% |
| Sorbitan laurate | 1.00% |
| Tocopherol | 0.20% |
| Propyl paraben | 0.10% |
| Disodium edetate | 0.10% |
| Glycerol 5.00% | |
| Allantoin | 0.20% |
| Acrylates/C₁₀₋₃₀ alkyl acrylate | |
| crosspolymer (Pemulen TR1) | 0.30% |
| Carbomer | 0.15% |
| Polysorbate 80 | 3.00% |
| Poloxamer 124 | 1.00% |
| Propylene glycol | 4.00% |
| Phenoxyethanol | 1.00% |
| Sodium hydroxide | qs pH 6.3-6.00 |
| Purified water | qs 100% |

15. Composition according to one of Claims 1 to 10, **characterized in that** it comprises:
| | |
|---|---|
| Ivermectin | 1.00% |
| Mineral oil | 10.00% |
| Sorbitan laurate | 1.00% |
| Tocopherol | 0.20% |
| Propyl paraben | 0.10% |
| Disodium edetate | 0.10% |
| Glycerol | 5.00% |
| Allantoin | 0.20% |
| Acrylates/C₁₀₋₃₀ alkyl acrylate | |
| crosspolymer (Pemulen TR1) | 0.30% |
| Carbomer | 0.15% |
| Polysorbate 80 | 4.00% |
| Propylene glycol | 4.00% |
| Benzyl alcohol | 3.00% |
| Sodium hydroxide | qs pH 6.3-6.00 |
| Purified water | qs 100% |

16. Composition according to one of Claims 1 to 10, **characterized in that** it comprises:
| | |
|---|---|
| Ivermectin | 0.03% |
| Mineral oil | 10.00% |
| Sorbitan laurate | 1.00% |
| Tocopherol | 0.20% |
| Propyl paraben | 0.10% |
| Disodium edetate | 0.10% |
| Glycerol | 5.00% |
| Allantoin | 0.20% |
| Acrylates/C₁₀₋₃₀ alkyl acrylate | |
| crosspolymer (Pemulen TR1) | 0.30% |
| Carbomer | 0.15% |
| Polysorbate 80 | 4.00% |
| Propylene glycol | 4.00% |
| Benzyl alcohol | 3.00% |
| Poly(methyl methacrylate) | 2.00% |
| Sodium hydroxide | qs pH 6.3-6.00 |
| Purified water | qs 100% |

17. Composition according to one of Claims 1 to 10, **characterized in that** it comprises:
| | |
|---|---|
| Ivermectin | 0.03% |
| Mineral oil | 5.00% |
| Sweet almond oil | 5.00% |
| Sorbitan laurate | 1.00% |
| Tocopherol | 0.20% |
| Propyl paraben | 0.10% |
| Disodium edetate | 0.10% |
| Glycerol | 5.00% |
| Allantoin | 0.20% |
| Acrylates/C₁₀₋₃₀ alkyl acrylate | |
| crosspolymer (Pemulen TR1) | 0.30% |
| Carbomer | 0.15% |
| Polysorbate 80 | 4.00% |
| Propylene glycol | 4.00% |
| Benzyl alcohol | 3.00% |
| Sodium hydroxide | qs pH 6.3-6.00 |
| Purified water | qs 100% |

18. Process for preparing, at room temperature, a composition according to one of Claims 1 to 17, comprising the following steps:
a) the constituents of the fatty phase are mixed until said phase is homogeneous;
b) the constituents of the aqueous phase are dissolved in water until complete homogeneity is obtained;
c) the polymeric emulsifier and the optional carbomer are dispersed in the aqueous phase until a homogeneous gel is obtained;
d) the fatty phase is incorporated, with moderate mechanical stirring, into the homogeneous gel obtained in c) so as to form an emulsion;
e) solvents and/or propenetrating agents for the active agent are mixed; the ivermectin is solubilized in this mixture; this mixture is then incorporated into the emulsion, with moderate mechanical stirring;
f) the neutralizing agent is incorporated, with moderate mechanical stirring, so as to obtain a defined pH.

19. Use of a composition according to one of Claims 1 to 17, for producing a pharmaceutical preparation intended to treat dermatological conditions, such as rosacea, common acne, seborrhoeic dermatitis, perioral dermatitis, acneform eruptions, transient acantholytic dermatitis, and acne miliaris necrotica.

20. Use according to Claim 19, **characterized in that** the pharmaceutical preparation is intended to treat rosacea.

## Patentansprüche

1. Pharmazeutische Zusammensetzung auf der Basis einer Verbindung der Avermectin-Gruppe in der Form einer Gel-Creme, die in einem physiologisch akzeptablen Medium eine Ölphase enthält, die mit Hilfe eines nicht grenzflächenaktiven, polymeren Emulgators in einer wässrigen Phase dispergiert ist, wobei die Ölphase Öle mit einem Schmelzpunkt unter 30 °C enthält und keine festen Fettsubstanzen mit einem Schmelzpunkt über 30 °C aufweist.

2. Pharmazeutische Zusammensetzung nach Anspruch 1, **dadurch gekennzeichnet, dass** die Verbindung der Avermectin-Gruppe unter Ivermectin, Invermectin, Avermectin, Abamectin, Doramectin, Eprinomectin und Selamectin ausgewählt ist.

3. Pharmazeutische Zusammensetzung nach einem der Ansprüche 1 oder 2, **dadurch gekennzeichnet, dass** es sich bei der Verbindung der Avermectin-Gruppe um Ivermectin handelt.

4. Zusammensetzung nach Anspruch 3, **dadurch gekennzeichnet, dass** sie enthält:
a) eine Ölphase, die aus Ölen mit einem Schmelzpunkt unter 30 °C zusammengesetzt ist und keine festen Fettsubstanzen mit einem Schmelzpunkt über 30 °C enthält;
b) einen nicht grenzflächenaktiven, polymeren Emulgator;
c) Ivermectin;
d) ein Lösungsmittel und/ oder einen penetrationsfördernden Stoff für den Wirkstoff;
e) und Wasser.

5. Zusammensetzung nach einem der Ansprüche 3 oder 4, **dadurch gekennzeichnet, dass** der Mengenanteil des Ivermectins 0,01 bis 10 Gew.-% und vorzugsweise 0,01 bis 5 Gew.-%, bezogen auf das Gesamtgewicht der Zusammensetzung, ausmacht.

6. Zusammensetzung nach einem der Ansprüche 1 bis 5, **dadurch gekennzeichnet, dass** der nicht grenzflächenaktive, polymere Emulgator unter Pemulen TR 1, Pemulen TR 2, Carbopol 1342, Carbopol 1382 und deren Gemischen ausgewählt ist.

7. Zusammensetzung nach Anspruch 4, **dadurch gekennzeichnet, dass** das Lösungsmittel und/oder der penetrationsfördernde Stoff unter Propylenglycol, Ethanol, Isopropanol, Butanol, Benzylalkohol, N-Methyl-2-pyrrolidon oder DMSO, Polysorbate 80, Phenoxyethanol und deren Gemischen ausgewählt ist.

8. Zusammensetzung nach einem der Ansprüche 1 bis 7, **dadurch gekennzeichnet, dass** sie Wasser in einem Mengenanteil im Bereich von 30 bis 95 Gew.-%, bezogen auf das Gesamtgewicht der Zusammensetzung, enthält.

9. Zusammensetzung nach einem der Ansprüche 1 bis 8, **dadurch gekennzeichnet, dass** sie enthält:
5 bis 20 % Ölphase;
0,25 bis 1 % nicht grenzflächenaktiven, polymeren Emulgator;
0 bis 2 % Carbomer,
0 bis 5 % Coemulgator,
0,01 bis 5 % Ivermectin,
0,1 bis 20 % Lösungsmittel und/oder penetrationsfördernde
Stoffe; und
60 bis 80 % gereinigtes Wasser.

10. Zusammensetzung nach einem der Ansprüche 1 bis 9, **dadurch gekennzeichnet, dass** sie enthält:
8 bis 15 % Ölphase;
0,25 bis 1 % nicht grenzflächenaktiven, polymeren Emulgator;
0 bis 2 % Carbomer,
0 bis 5 % Coemulgator,
0,01 bis 5 % Ivermectin,
4 bis 20 % Lösungsmittel und/ oder penetrationsfördernde Stoffe; und
60 bis 80 % gereinigtes Wasser.

11. Zusammensetzung nach einem der Ansprüche 1 bis 10, **dadurch gekennzeichnet, dass** sie enthält:
| | |
|---|---|
| Ivermectin | 1,00 % |
| Mineralöl | 10,00 % |
| Tocopherol | 0,20 % |
| Propylparaben | 0,10 % |
| Dinatriumedetat | 0,10 % |
| Glycerol | 5,00 % |
| Allantoin | 0,20 % |
| Acrylates/C₁₀₋₃₀-Alkylacrylate Crosspolymer | 0,30 % |
| Carbomer | 0,15 % |
| Polysorbate 80 | 4,00 % |
| Propylenglycol | 4,00 % |
| Phenoxyethanol | 1,00 % |
| Natriumhydroxid | qs pH 6,30 |
| Gereinigtes Wasser | qs 100 |

12. Zusammensetzung nach einem der Ansprüche 1 bis 10, **dadurch gekennzeichnet, dass** sie enthält:
| | |
|---|---|
| Ivermectin | 1,00 % |
| Mineralöl | 10,00 % |
| Tocopherol | 0,20 % |
| Dinatriumedetat | 0,10 % |
| Glycerol | 5,00 % |
| Acrylates/C₁₀₋₃₀-Alkylacrylate Crosspolymer | 0,30 % |
| Carbomer | 0,15 % |
| Polysorbate 80 | 4,00 % |
| Propylenglycol | 4,00 % |
| Phenoxyethanol | 1,00 % |
| Triethanolamin | qs pH 6,00 |
| Gereinigtes Wasser | qs 100 % |

13. Zusammensetzung nach einem der Ansprüche 1 bis 10, **dadurch gekennzeichnet, dass** sie enthält:
| | |
|---|---|
| Ivermectin | 0, 50 % |
| Capryl/Caprinsäuretriglyceride | 10,00 % |
| Tocopherol | 0,20 % |
| Dinatriumedetat | 0,10 % |
| Sorbitol | 5,00 % |
| Zinkacetat | 0,50 % |
| Acrylates/C₁₀₋₃₀-Alkylacrylate Crosspolymer | 0,30 % |
| Carbomer | 0,15 % |
| N-Methylpyrrolidon | 5,00 % |
| Phenoxyethanol | 1,00 % |
| Natriumhydroxid | qs pH 6,00 |
| Gereinigtes Wasser | qs 100 % |

14. Zusammensetzung nach einem der Ansprüche 1 bis 10, **dadurch gekennzeichnet, dass** sie enthält:
| | |
|---|---|
| Ivermectin | 1,00 % |
| Mineralöl | 10,00 % |
| Sorbitanlaurat | 1,00 % |
| Tocopherol | 0,20 % |
| Propylparaben | 0,10 % |
| Dinatriumedetat | 0,10% |
| Glycerol | 5,00 % |
| Allantoin | 0,20 % |
| Acrylates/C₁₀₋₃₀-Alkylacrylate Crosspolymer | 0,30 % |
| (Pemulen TR1) | |
| Carbomer | 0,15 % |
| Polysorbate 80 | 3,00 % |
| Poloxamer 124 | 1,00 % |
| Propylenglycol | 4,00 % |
| Phenoxyethanol | 1,00 % |
| Natriumhydroxid | qs pH 6,3-6,00 |
| Gereinigtes Wasser | qs 100 % |

15. Zusammensetzung nach einem der Ansprüche 1 bis 10, **dadurch gekennzeichnet, dass** sie enthält:
| | |
|---|---|
| Ivermectin | 1,00 % |
| Mineralöl | 10,00 % |
| Sorbitanlaurat | 1,00 % |
| Tocopherol | 0,20 % |
| Propylparaben | 0,10% |
| Dinatriumedetat | 0,10 % |
| Glycerol | 5,00 % |
| Allantoin | 0,20 % |
| Acrylates/C₁₀₋₃₀-Alkylacrylate Crosspolymer | 0,30 % |
| (Pemulen TR1) | |
| Carbomer | 0,15 % |
| Polysorbate 80 | 4,00 % |
| Propylenglycol | 4,00 % |
| Benzylalkohol | 3,00 % |
| Natriumhydroxid | qs pH 6,3-6,00 |
| Gereinigtes Wasser | qs 100 % |

16. Zusammensetzung nach einem der Ansprüche 1 bis 10, **dadurch gekennzeichnet, dass** sie enthält:
| | |
|---|---|
| Ivermectin | 0,03 % |
| Mineralöl | 10,00 % |
| Sorbitanlaurat | 1,00 % |
| Tocopherol | 0,20 % |
| Propylparaben | 0,10 % |
| Dinatriumedetat | 0,10 % |
| Glycerol | 5,00 % |
| Allantoin | 0,20 % |
| Acrylates/C₁₀₋₃₀-Alkylacrylate Crosspolymer | 0,30 % |
| (Pemulen TR1) | |
| Carbomer | 0,15 % |
| Polysorbate 80 | 4,00 % |
| Propylenglycol | 4,00 % |
| Benzylalkohol | 3,00 % |
| Poly(methylmethacrylat) | 2,00 % |
| Natriumhydroxid | qs pH 6,3-6,00 |
| Gereinigtes Wasser | qs 100 % |

17. Zusammensetzung nach einem der Ansprüche 1 bis 10, **dadurch gekennzeichnet, dass** sie enthält:
| | |
|---|---|
| Ivermectin | 0,03 % |
| Mineralöl | 5,00 % |
| Süßmandelöl | 5,00 % |
| Sorbitanlaurat | 1,00 % |
| Tocopherol | 0,20 % |
| Propylparaben | 0,10 % |
| Dinatriumedetat | 0,10% |
| Glycerol | 5,00 % |
| Allantoin | 0,20 % |
| Acrylates/C₁₀₋₃₀-Alkylacrylate Crosspolymer | 0,30 % |
| (Pemulen TR1) | |
| Carbomer | 0,15 % |
| Polysorbate 80 | 4,00 % |
| Propylenglycol | 4,00 % |
| Benzylalkohol | 3,00 % |
| Natriumhydroxid | qs pH 6,3-6,00 |
| Gereinigtes Wasser | qs 100 % |

18. Verfahren zur Herstellung einer Zusammensetzung nach einem der Ansprüche 1 bis 17 bei Raumtemperatur umfassend die folgenden Schritte:
a) die Bestandteile der Fettphase werden bis zur vollständigen Homogenität dieser Phase vermischt;
b) die Bestandteile der wässrigen Phase werden bis zur vollständigen Homogenität in Wasser gelöst;
c) der polymere Emulgator und das optionale Carbomer werden in der wässrigen Phase dispergiert, bis ein homogenes Gel erhalten ist;
d) die Fettphase wird zur Bildung einer Emulsion unter mäßigem mechanischen Rühren in das in Schritt c) gebildete homogene Gel eingebracht;
e) die Lösungsmittel und/oder penetrationsfördernden Stoffe für den Wirkstoff werden vermischt; das Ivermectin wird in dem Gemisch gelöst; dieses Gemisch wird anschließend unter mäßigem mechanischen Rühren in die Emulsion eingebracht;
f) das Neutralisationsmittel wird zur Einstellung eines definierten pH-Wertes unter mäßigem mechanischen Rühren hinzugefügt.

19. Verwendung einer Zusammensetzung nach einem der Ansprüche 1 bis 17 für die Herstellung einer pharmazeutischen Zusammensetzung, die für die Behandlung dermatologischer Zustände vorgesehen ist, wie Rosacea, Acne vulgaris, seborrhoische Dermatitis, periorale Dermatitis, acneiformes Exanthem, transiente akantholytische Dermatose und Acne miliaris necrotica.

20. Verwendung nach Anspruch 19, **dadurch gekennzeichnet, dass** die pharmazeutische Zubereitung für die Behandlung von Rosacea vorgesehen ist.

## Revendications

1. Composition pharmaceutique basée sur un composé de la famille de l'avermectine, sous la forme d'une crème-gel comprenant, dans un milieu physiologiquement acceptable, une phase huileuse dispersée dans une phase aqueuse au moyen d'un émulsifiant polymère non tensioactif, ladite phase huileuse comprenant des huiles ayant un point de fusion au-dessous de 30 °C et étant exempte de graisses solides ayant un point de fusion au-dessus de 30 °C.

2. Composition pharmaceutique selon la revendication 1, **caractérisée en ce que** le composé de la famille de l'avermectine est choisi parmi l'ivermectine, l'invermectine, l'avermectine, l'abamectine, la doramectine, l'éprinomectine et la sélamectine.

3. Composition pharmaceutique selon l'une des revendications 1 et 2, **caractérisée en ce que** le composé de la famille de l'avermectine est l'ivermectine.

4. Composition selon la revendication 3, **caractérisée en ce qu'**elle comprend :
a) une phase huileuse composée d'huiles ayant un point de fusion au-dessous de 30 °C et étant exempte de graisses solides ayant un point de fusion au-dessus de 30 °C,
b) un émulsifiant polymère non tensioactif,
c) de l'ivermectine,
d) un solvant et/ou un agent propénétrant pour l'agent actif,
e) et de l'eau.

5. Composition selon l'une des revendications 3 et 4, **caractérisée en ce que** la quantité d'ivermectine représente de 0,01 à 10 %, et de préférence de 0,01 à 5 %, en poids par rapport au poids total de la composition.

6. Composition selon l'une des revendications 1 à 5, **caractérisée en ce que** l'émulsifiant polymère non tensioactif est choisi parmi Pemulen TR 1, Pemulen TR 2, Carbopol 1342, Carbopol 1382 ou des mélanges de ceux-ci.

7. Composition selon la revendication 4, **caractérisée en ce que** le solvant et/ou l'agent propénétrant sont choisis parmi le propylèneglycol, l'éthanol, l'isopropanol, le butanol, l'alcool benzylique, la N-méthyl-2-pyrrolidone ou le DMSO, le polysorbate 80, le phénoxyéthanol, et des mélanges de ceux-ci.

8. Composition selon l'une des revendications 1 à 7, **caractérisée en ce qu'**elle contient une quantité d'eau dans la plage de 30 à 95 % en poids par rapport au poids total de la composition.

9. Composition selon l'une des revendications 1 à 8, **caractérisée en ce qu'**elle comprend :
de 5 à 20 % de phase huileuse,
de 0,25 à 1 % d'émulsifiant polymère non tensioactif,
de 0 à 2 % de carbomère,
de 0 à 5 % de co-émulsifiant,
de 0,01 à 5 % d'ivermectine,
de 0,1 à 20 % de solvants et/ou d'agents propénétrants,
de 60 à 80 % d'eau purifiée.

10. Composition selon l'une des revendications 1 à 9, **caractérisée en ce qu'**elle comprend :
de 8 à 15 % de phase huileuse,
de 0,25 à 1 % d'émulsifiant polymère non tensioactif,
de 0 à 2 % de carbomère,
de 1 à 5 % de co-émulsifiant,
de 0,01 à 5 % d'ivermectine,
de 4 à 20 % de solvants et/ou d'agents propénétrants,
de 60 à 80 % d'eau purifiée.

11. Composition selon l'une des revendications 1 à 10, **caractérisée en ce qu'**elle comprend :
| | |
|---|---|
| Ivermectine | 1,00 % |
| Huile minérale | 10,00 % |
| Tocophérol | 0,20 % |
| Propylparabène | 0,10 % |
| Édétate disodique | 0,10 % |
| Glycérol | 5,00 % |
| Allantoïne | 0,20 % |
| Polymère croisé d'acrylates/acrylate | |
| d'alkyle en C₁₀₋₃₀ | 0,30 % |
| Carbomère | 0,15 % |
| Polysorbate 80 | 4,00 % |
| Propylèneglycol | 4,00 % |
| Phénoxyéthanol | 1,00 % |
| Hydroxyde de sodium | q.s.p. pH 6,30 |
| Eau purifiée | q.s.p. 100 |

12. Composition selon l'une des revendications 1 à 10, **caractérisée en ce qu'**elle comprend :
| | |
|---|---|
| Ivermectine | 1,00 % |
| Huile minérale | 10,00 % |
| Tocophérol | 0,20 % |
| Édétate disodique | 0,10 % |
| Glycérol | 5,00 % |
| Polymère croisé d'acrylates/acrylate | |
| d'alkyle en C₁₀₋₃₀ | 0,30 % |
| Carbomère | 0,15 % |
| Polysorbate 80 | 4,00 % |
| Propylèneglycol | 4,00 % |
| Phénoxyéthanol | 1,00 % |
| Triéthanolamine | q.s.p. pH 6,00 |
| Eau purifiée | q.s.p. 100 %. |

13. Composition selon l'une des revendications 1 à 10, **caractérisée en ce qu'**elle comprend :
| | |
|---|---|
| Ivermectine | 0,50 % |
| Triglycérides capriques/capryliques | 10,00 % |
| Tocophérol | 0,20 % |
| Édétate disodique | 0,10 % |
| Sorbitol | 5,00 % |
| Acétate de zinc | 0,50 % |
| Polymère croisé d'acrylates/acrylate | |
| d'alkyle en C₁₀₋₃₀ | 0,30 % |
| Carbomère | 0,15 % |
| N-méthylpyrrolidone | 5,00 % |
| Phénoxyéthanol | 1,00 % |
| Hydroxyde de sodium | q.s.p. pH 6,00 |
| Eau purifiée | q.s.p. 100 %. |

14. Composition selon l'une des revendications 1 à 10, **caractérisée en ce qu'**elle comprend :
| | |
|---|---|
| Ivermectine | 1,00 % |
| Huile minérale | 10,00 % |
| Laurate de sorbitan | 1,00 % |
| Tocophérol | 0,20 % |
| Propylparabène | 0,10 % |
| Édétate disodique | 0,10 % |
| Glycérol | 5,00 % |
| Allantoïne | 0,20 % |
| Polymère croisé d'acrylates/acrylate | |
| d'alkyle en C₁₀₋₃₀ (Pemulen TR1) | 0,30 % |
| Carbomère | 0,15 % |
| Polysorbate 80 | 3,00 % |
| Poloxamer 124 | 1,00 % |
| Propylèneglycol | 4,00 % |
| Phénoxyéthanol | 1,00 % |
| Hydroxyde de sodium | q.s.p. pH 6,3-6,00 |
| Eau purifiée | q.s.p. 100 %. |

15. Composition selon l'une des revendications 1 à 10, **caractérisée en ce qu'**elle comprend :
| | |
|---|---|
| Ivermectine | 1,00 % |
| Huile minérale | 10,00 % |
| Laurate de sorbitan | 1,00 % |
| Tocophérol | 0,20 % |
| Propylparabène | 0,10 % |
| Édétate disodique | 0,10 % |
| Glycérol | 5,00 % |
| Allantoïne | 0,20 % |
| Polymère croisé d'acrylates/acrylate | |
| d'alkyle en C₁₀₋₃₀ (Pemulen TR1) | 0,30 % |
| Carbomère | 0,15 % |
| Polysorbate 80 | 4,00 % |
| Propylèneglycol | 4,00 % |
| Alcool benzylique | 3,00 % |
| Hydroxyde de sodium q.s.p. pH 6,3-6,00 | |
| Eau purifiée | q.s.p. 100 %. |

16. Composition selon l'une des revendications 1 à 10, **caractérisée en ce qu'**elle comprend :
| | |
|---|---|
| Ivermectine | 0,03 % |
| Huile minérale | 10,00 % |
| Laurate de sorbitan | 1,00 % |
| Tocophérol | 0,20 % |
| Propylparabène | 0,10 % |
| Édétate disodique | 0,10 % |
| Glycérol | 5,00 % |
| Allantoïne | 0,20 % |
| Polymère croisé d'acrylates/acrylate | |
| d'alkyle en C₁₀₋₃₀ (Pemulen TR1) | 0,30 % |
| Carbomère | 0,15 % |
| Polysorbate 80 | 4,00 % |
| Propylèneglycol | 4,00 % |
| Alcool benzylique | 3,00 % |
| Poly(méthacrylate de méthyle) | 2,00 % |
| Hydroxyde de sodium | q.s.p. pH 6,3-6,00 |
| Eau purifiée | q.s.p. 100 %. |

17. Composition selon l'une des revendications 1 à 10, **caractérisée en ce qu'**elle comprend :
| | |
|---|---|
| Ivermectine | 0,03 % |
| Huile minérale | 5,00 % |
| Huile d'amande douce | 5,00 % |
| Laurate de sorbitan | 1,00 % |
| Tocophérol | 0,20 % |
| Propylparabène | 0,10 % |
| Édétate disodique | 0,10 % |
| Glycérol | 5,00 % |
| Allantoïne | 0,20 % |
| Polymère croisé d'acrylates/acrylate | |
| d'alkyle en C₁₀₋₃₀ (Pemulen TR1) | 0,30 % |
| Carbomère | 0,15 % |
| Polysorbate 80 | 4,00 % |
| Propylèneglycol | 4,00 % |
| Alcool benzylique | 3,00 % |
| Hydroxyde de sodium | q.s.p. pH 6,3-6,00 |
| Eau purifiée | q.s.p. 100 %. |

18. Procédé de préparation, à température ambiante, d'une composition selon l'une des revendications 1 à 17, comprenant les étapes suivantes :
a) les constituants de la phase grasse sont mélangés jusqu'à ce que ladite phase soit homogène ;
b) les constituants de la phase aqueuse sont dissous dans de l'eau jusqu'à ce qu'une homogénéité totale soit obtenue ;
c) l'émulsifiant polymère et le carbomère éventuel sont dispersés dans la phase aqueuse jusqu'à ce qu'un gel homogène soit obtenu ;
d) la phase grasse est incorporée, sous agitation mécanique modérée, dans le gel homogène obtenu dans c) de manière à former une émulsion ;
e) les solvants et/ou les agents propénétrants pour l'agent actif sont mélangés ; l'ivermectine est solubilisée dans ce mélange ; ce mélange est ensuite incorporé dans l'émulsion, sous agitation mécanique modérée ;
f) l'agent neutralisant est incorporé, sous agitation mécanique modérée, de manière à obtenir un pH défini.

19. Utilisation d'une composition selon l'une des revendications 1 à 17 pour produire une préparation pharmaceutique destinée à traiter des affections dermatologiques, telles que l'acné rosacée, l'acné commune, la parakétose séborrhéïque, la dermatite périorale, les éruptions acnéiformes, la dermatose acantholytique transitoire, et l'acné miliaire nécrotique.

20. Utilisation selon la revendication 19, **caractérisée en ce que** la préparation pharmaceutique est destinée à traiter l'acné rosacée.
